Europäisches Patentamt
European Patent Office
Office européen des brevets

(11) Publication number: **0 053 681**
**A1**

(12) **EUROPEAN PATENT APPLICATION**

(21) Application number: 81108526.5

(22) Date of filing: 20.10.81

(51) Int. Cl.³: C 07 D 213/80
C 07 D 213/83, A 61 K 31/455

(30) Priority: 21.10.80 IT 4520880

(43) Date of publication of application:
16.06.82 Bulletin 82/24

(84) Designated Contracting States:
AT BE CH DE GB LI LU NL SE

(71) Applicant: Tauro, Celesio Sonnino
Via Spinello Aretino No.6
I-56100 Pisa(IT)

(72) Inventor: Tauro, Celesio Sonnino
Via Spinello Aretino No.6
I-56100 Pisa(IT)

(54) **(Bis-thio-ethyl-nicotinate)-1,10-decane, new hypolipidemic substance and various methods of procedure.**

(57) The present invention is referred to a proceeding of preparation to obtain original derivatives from the nicotinic acid and in particular thioethers, thioesters from the formula:

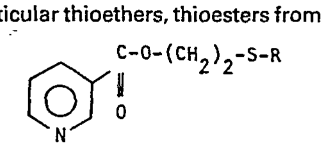

in which R may signify:

a) - an acrylic group of the kind: 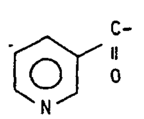

b) - an acylalchil of the kind: 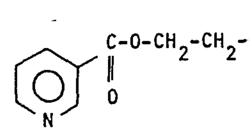

c) - an acylalchilthio of the general formula connected with a methylenic group with a varied number of C athoms:

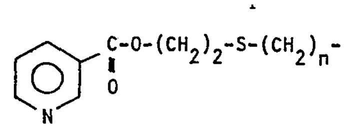

When as in case "c", a methylenic group is represented by n = 10 we obtain a new compound: 2-(decamethylendithio)-2'-diethyl-dinicotinate answering to the following structural formula with properties and characteristics well defined:

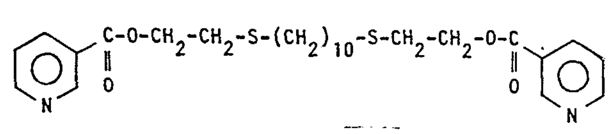

This new compound may be considered a derivative of 2,2'-(decamethylen-dithio)-dietanol, known in therapy as hypolipidemic in which the alcoholic groups are hesterified with two moles of Nicotinic acid.

The product obtained:(BIS-THIO-HETHYL-NICOTINATE)-1,10-DE=CANE is an original substance of synthesis provided with a very clear normalizing action in the altered lipidic methabolism of atherosclerotic subject.

EP 0 053 681 A1

NEW HYPOLIPIDEMIC SUBSTANCE:

(BIS-THIO-HETHYL-NICOTINATE)-1,10-DECANE

TAURO CELESIO SONNINO Italy - Pisa - 6 Spinello Aretino

This invention refers to a process of preparation in order to obtain original derivatives from the nicotinic acid and in particular thioethers, thioesters with the formula:

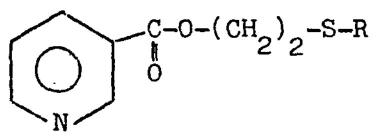

where R may signify:

a) - an acylic group of the kind:

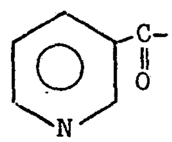

b) - an acylalchil of the kind:

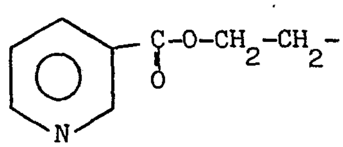

c) - an acylalchilthio of the general formula connected with a methylenic group, with a varied number of C atoms:

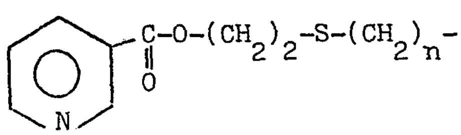

When-as in case c- a methylenic group is represented by n=10, we obtein a new compound:2-(decamethylendithio)-2'-diethyl-di=nicotinate, answering to the follcwing structural formula with properties and characteristics well defined:

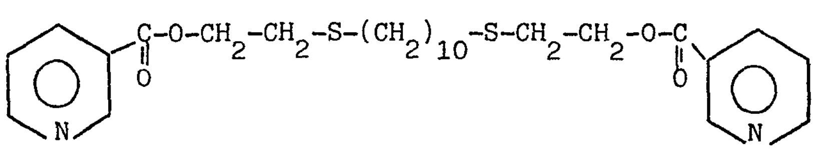

This new compound may be considered a derivative of 2.2'-(de=camethylen-dithio)-dietanol, known in therapy as a hypolipidemic in which the alcoholic groups are hesterified with two moles of nicotinic acid. It can be obteined from the two compounds , reacted in presence of cathalitic agents and solvents.

DESCRIPTIVE MEMORY

The product obtained (BIS-THIO-HETHYL-NICOTINATE)-1.10-DECANE is an original substance of synthesis provided with a very clear normalizing action in the altered lipidic methabolism of arteriosclerotic subjects.

This compound has a therapeutical action, such to reduce the haematic rate of total lipids, of triglicerids and of cholesterol in cases of hyperlipidemy.

The hesterification of nicotinic acid with Bis (hydroxy-2-e=thylthyo)-1,10-decane increases and extends the terapeutical action, given by the single compounds, thanks to the slow release of nicotinic acid, which presents some collateral effects: prurigo, hot flushes, when absorbed rapidly.

Recently nicotinic acid has been discovered to present also profibrinolitic and hypolipidemic action.

Profibrinolitic action seems to be connected with the capability of nicotinic acid of facilitating the release of an activator of plasminogen of vases endotheluine and/or slackening

the hepatic clearance of the plasminogen.

Hypolipidemic activity follows the inhibition of cholesterol synthesis in liver and of mobilization of NEFA from adipose tissue; for antilipolitc action, the plasmatic levels of NEFA decrease and the synthesis of triglicerids reduces.

The reduction of NEFA might influence the aggregation of piastrine; in fact it is well-known the iperaggregating action of free fat acids, as well as pathogenic implications of augmented aggregation of piastrine in artheriosclerosis.

The present product frees nicotinic acid and Bis (hydroxy-2--ethyltio)-1,10-decane very slowly, increasing and extending the therapeutical activity.

Pharmacological and clinical studies have shown the hypolipidemic activity of this new substance, its easy absorption, its affinity to plasmatic patients, its quick metabolization at hepatic level, through a procedure of oxidation and glucurono--sulfoconiugation, by means of sulfonilic groups.

The expulsion is above all through the renal emuntory and feces within 48 hrs. from assumption, avoiding any accumulation of the drug in the various organs.

The therapeutical activity of the new substance reveals inhibition of LDL and VLDL (both atherogen lypoproteins) and leaves HDL (antiatherogen lypoproteins) unhaltered, and so it

modifies positively the atherogenetic index (IA):

$$\frac{VLDL + LDL}{HDL} = IA$$, with the advantage of reducing the chief risk factor of arteriosclerotic pathology.

Administrating the new mulecule, with equal doses of the single compounds above mentioned, we obtain a better respondence of the therapeutic margins, in respect to $DL_{50}$, when administrated orally.

Pharmacological proofs on mice showed a very good pharmacodinamic activity on lowering triglicerids, lipids and cholesterol, superior to 2.2'-(decamethyl-dithio)-diethanol and to nicotinic acid and other compounds on sale.

It was impossible to determine $DL_{50}$ in acute toxicity because any sympton of intolerability is not noticeable even at high doses.

The new compound can be administered orally in its different forms (tablets, capsules, etc.) and by hypodermic injection, when necessary.

The product is obtained (Method A) by reaction of nicotineilchloride hydrochloride with 1.10 (bis-hydroxyethyl-thio)-decane in presence of an acceptor of hydrochloric acid, such as pyridine, triethilamine, N.N-Dimethylaniline, etc. It can be also obtained according to method B by reaction of anhidride of nicotinic acid with 1.10-(bis-hydroxyethyl-thio)-decane (U.S.P. 3,021,215) in presence of Pyridine.

According to Method C the product was obtained by reaction of nicotinic acid chlorure with 2-Mercaptoethanol (II) and the product 2-Mercaptoethylnicotinate furtherly reacted with 1.10-Dibromodecane in presence of sodium methyle (or sodium hydroxide). We obtained the same product as in Method A and B, that is 2-(Decamethylendithio)-2'-diethyl-dinicotinate.

2-Mercaptoethylnicotinate (III) can react with 1,9 Decadiene (Method D) in presence of 360 Watt mercury lamp, 20 cm distant, for about 2 hrs.

The following scheme shows the above mentioned Methods.

SYNTHESIS SCHEME

METHOD A:

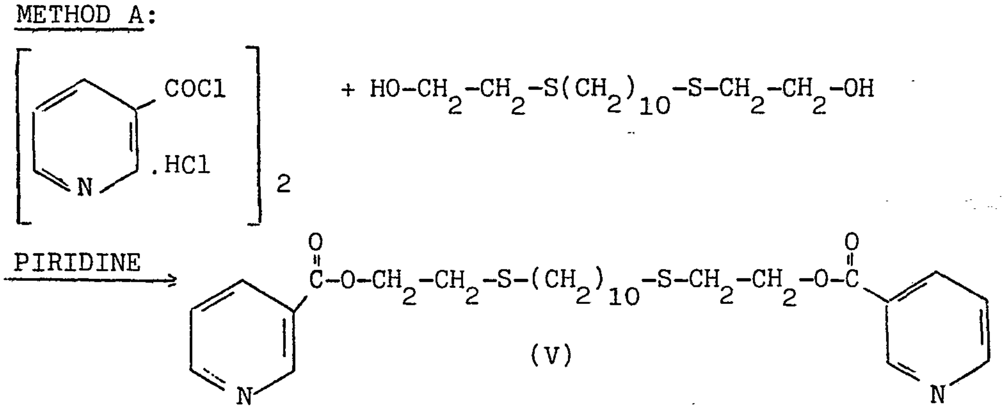


(V)

METHOD B:

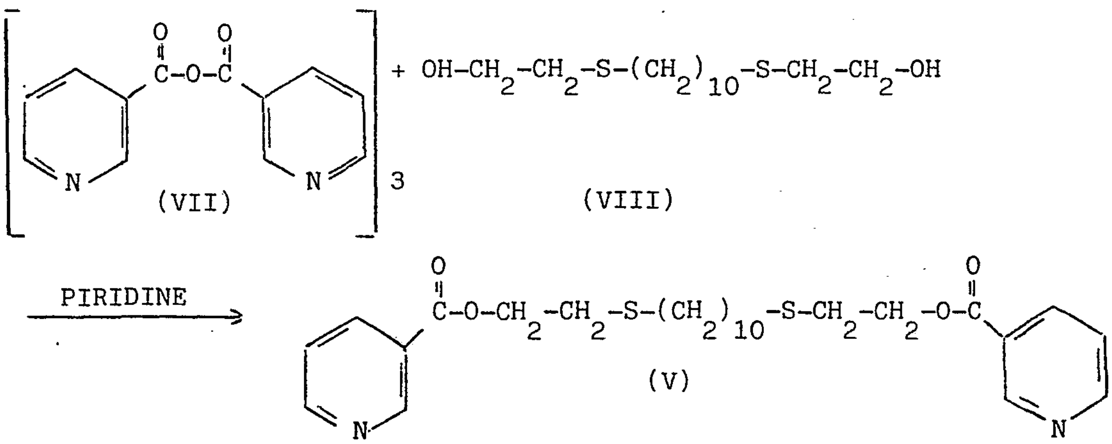


(VII) (VIII)

(V)

METHOD C:

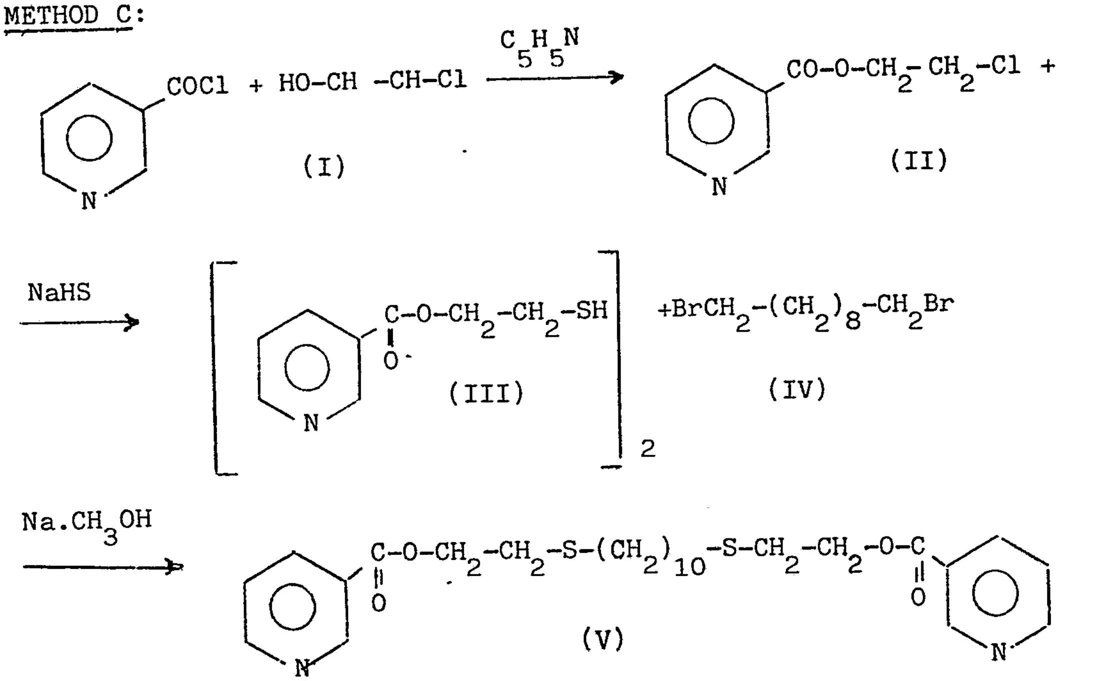


METHOD D:

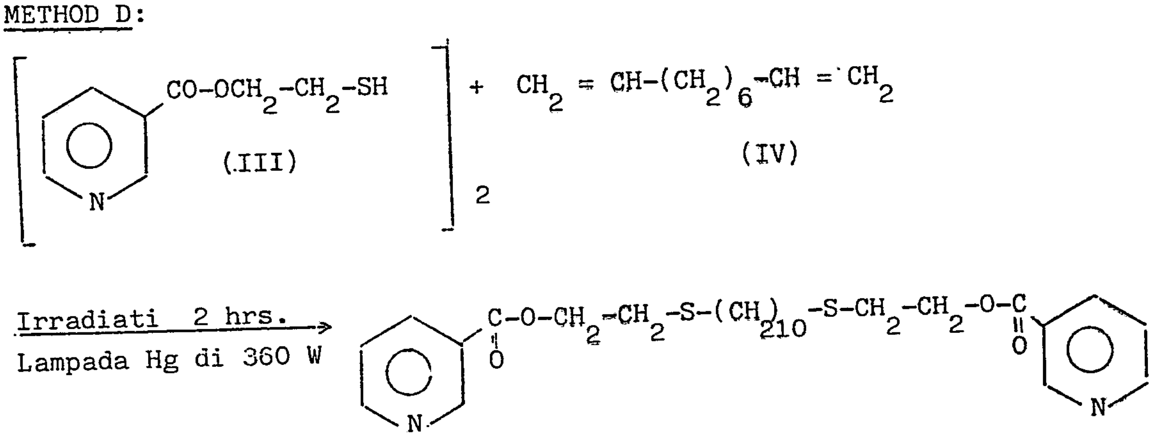


METHOD A:

PREPARATION OF 2,2'-(DECAMETHYLENDITHIO)-DIETHYL-DINICOTINATE (V):

$C_{26}H_{36}N_2O_4S_2$ P.M. = 504.70

Put gr.147 (0.5 mole) of (bis-hydroxyethyl-thio) -1.10 decane and 350 ml of chloroform anhydre in a three- necks flask with mechanical mixer, refluxing with $CaCl_2$ pipe, dropping funnel and thermo=

mether placed in thermoregulable bath.

Add to the solution (1,15 mole) gr.205 of nicotinoilchlorure hydrochloride freshly prepared.

The mixture is stirred while adding gr.205 (2,6 mole) of anhydre piridine, through the drip funnel. The addition of piridine is adjusted in order to keep exothermic reaction temperature at about 65°C, to mantein a light reflux. Reflux is kept on by heating for 3 hrs. and then reaction mixture (pH 3,8-4,2) is cooled to crystallize and put into refrigerator at 5°C for 20 hrs. with desultory stirring.

The white crystalline product is collected on funnel, well squeezed, washed with a little cold chloroform and all the filtered is put aside with the washings.

The product on funnel is washed with water and sodium bicarbonate solution and then distilled water, up to complete elimination of chlorides.

The product, dried at 50°C in vacuum, presents white and crystalline, weighs about 158 gr with m.p. 70-71° in Kofler.

The chloroformic solution is washed with water to eliminate pyridine hydrochloride, with solution of sodium bicarbonate and moreover with distilled water.

The chloroformic solution is distilled to remove the solvent and the residual is dissolved with water, collected on funnel,

washed with distilled water and the product dried at 50°C in vacuum, gives gr.51 of product with m.p. 70-71°C.

Total gr.209. Yield 83% of theoricum.

Purification

The product purified with ethyl alcohol ml 9/gr. of product gives bright white lamellae with m.p. 71°C. Yield 80%.

Centesimal analysis

For $C_{26}H_{36}N_2O_4S_2$ Found.%: C=62.19; H=7.09; N=5.52; S=12.75

Calculated.%:C=61.88;H=7.19; N=5.55; S=12.70

METHOD B:

1) Nicotinic anhydride (M.W.228)(0.3 mole) ..........gr. 68.4

2) Pyridine anhydrous (0.5 mole) ..................ml. 40.-

3) (Bis-hydroxiethyl-thio)-1.10 decane (0.1 mole)....gr. 29.4

Put the compounds 1) 2) and 3) in a three necks flask with $CaCl_2$ drier pipe. The mixture is stirred and heated on water bath for 4 hrs. The product is poured and stirred in 350 ml of water and alkalinized with watery solution saturated with sodium carbonate.

The oily layer is extracted with three portions of ml.140 chloroform or methylene chloride.

The extracts are washed several times with water, dehydrated with sodium sulphate anhydrous, filtered and the solvent removed by distillation with traces of residual pyridine.

The residual oil still hot crystallizes by cooling with an almost theoretic yield. We obtein a white product, melting at 68-69°C.

Recrystallized with ethyl alcohol, m.p.70-71°C

The product obteined is exactly alike to one of Method A) (the same product is obteined by reaction of nicotinic acid with (bis--hydroxy-ethylthio)-1,10 decane in presence of Duolite Resine C - 20, or correspondent to other type of strong cationic resine at appropriate temperature).

METHOD C:

2-CHLOROETHYL-NICOTINATE (II)

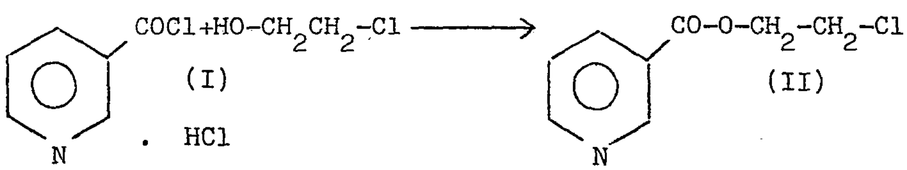

(I) (II)

$C_8H_8ClNO_2$

P.M. = 185.62

b.p.$_{0.4-0.5}$= 88-90°

1)Nicotinoylchloride hydrochlorid (0.5 mole) .........gr. 89.-

2)Chloroethanol (0.5 mole) ..........................gr.40.25

3)Chloroform anhydrous ..............................ml 150

4)Pyridine anhydrous (0.5 mole) .....................gr.39.5

Put the compounds 1)2) and 3) in a three necks flask with mechanical mixer, refluxing refrigerator with $CaCl_2$ pipe, the stirring

mixture heated on water bath begins to react developing HCl. The reaction finishes with slow addition of 4) and continuing the reflux on water bath for 3 hrs. more.

After cooling the mixture is beated with water, the organic phase separated, washed with solution of Sodium bicarbonate until it results neutral, then it is washed with water again and dehydrated on sodium sulphate anhydrous.

The solvent is removed from the filtered and gives a residual of gr.84.

The product subjeted to fractionation at reduced pressure boils at b.p.$_{0.4-0.5}$ 88-90°C or at$_{0.7\ mm}$ 93°C.

The product presents like colourless oil yield gr. 65.7 (70% of calculated).

The distilled colourless product crystallizes by cooling in bright crystals and melts at 28°C.

2-Chloroethyl-nicotinate (II) is very irritant, in contact with skin.

Analysis:

For $C_8H_8ClNO_2$ Found.%: C=51.77; H=4.35; Cl=19.10; N=7.54

Calculated.%: C=51.50; H=4.70; Cl=19.42; N=7.81

2-MERCAPTOETHYL-NICOTINATE (III)

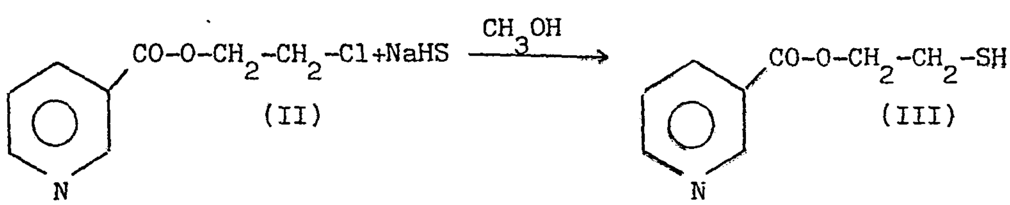


(II) (III)

1) 2-Chloroethyl-nicotinate (0.1 mole) ..............gr. 18.6

2) Absolute methanole ...............................ml. 200

3) Sodium hydrosulphite (sodium bisulphite)(0.5 mole) gr. 6.12

The mixture of compounds 1)2) and 3) is stirred and heated on water bath up to complete reaction. After a night of rest, the solvent is removed at reduced pressure on water bath and the residue is dissolved with chloroform, the chloroformic solution is washed several times with water, dehydrated an $Na_2SO_4$;the solvent is ditilled and the residual fractionated at reduced pressure, boils at b.p.$_{0.8}$=113-116°;liquid colourless oily or ligthly straw-coloured.

Analysis:

For $C_8H_9NO_2S$ Found %:C=52.44; H=4.95; N=7.65; S=17.50

Calculated %:C=52.51;H=5.02; N=7.62; S=17.53

The produit in contact with skin causes redness and vasodilatation. 2-Mercaptoethylnicotinate can be obteined also from nicotinic acid with 2-Mercapto-ethanole in presence for Duolite C-20 resin or other correspondent resin. The same product is obteined by reaction of nicotinoylchloride-hydrochloride with 2-Mercaptoethanole according to the following scheme:

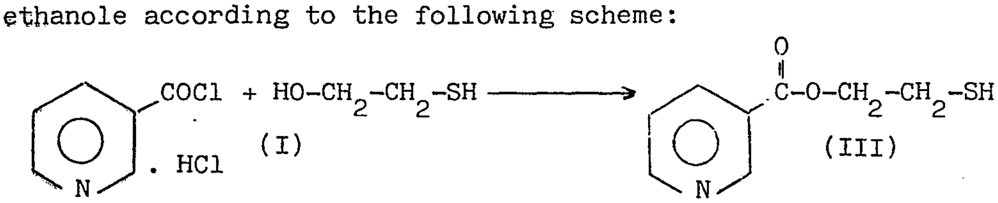

(I) (III)

1) Nicotinoylchlorure hydrochloride (0.5 mole) .........gr 89

2) 2-Mercaptoethanole (0.5 mole) .............ml 35 gr 39

3) Chloroform anhydrous ................................ ml 150

Put the compounds 1) and 2) in a three necks flask provided with refluxing refrigerator with $CaCl_2$ pipe, mechanic mixer, dropping funnel placed in thermoregulable bath. The stirring mixture negins to react, developing heat and gas HCl as 3) is added, until it reaches the reflux temperature of the solvent. When the addition finishes the reflux is continued on water bath for 3 hrs. After cooling, the mixture is washed with 300 ml of water and neutralized adding sodium bicarbonate.The chloroformic phase is separated, washed several times with wather, dehydrated on $Na_2SO_4$, filtered and the solvent is evaporated,obteining a residual amounting to gr.79 (86.8%).

After distillation at reduced pressure we obtein gr.65 (70% of calculated) at b.p.$_{1.5}$=132-135° or at$_{0.8\ mm}$113-116°C.

From distillation we have a residual of gr.6.2 (6.7%) wich crystallizes by cooling and purifying it with isopropanole we obtein white soft crystals melting at 82-83°C,identified with Nicotinylthio-ethylnicotinate of the following structure:

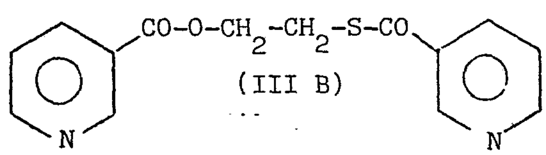

(III B)

and with periferical vasodilatating property.

Analysis:

$C_{14}H_{12}N_2O_3S$ Found %: C=58.32; H=4.19; N=9.71; S=11.12

Calculated %: C=58.38; H=4.20; N=9.51; S=11.17

When in the reaction 2 moles of nicotinylchlorure and 1 mole of 2-Mercaptoethanole are used in presence of pyridine, the reactions goes on in favour of nicotinilthio-ethylnicotinate (III B) with high yields.

(BIS-THIOETHYLNICOTINATE)-1.10 DECANE (V)

METHOD C:

Absolute methanole ..............................ml 500

Metallic Sodium (0.5 gr.at.) .....................gr 11.5

2) 2-Mercaptoethylnicotinate (III)(0.5 mole) .........gr 92

3) 1.10-Dibromodecane (0.25 mole). ...................gr 75

Sodium is dissolved in absolute methanole and 2) is added to cold solution of sodium methylate.

3) is then added little by little to mixture of reaction at room temperature, while stirring and stirred for some hrs. and then let to rest all the night long.

Methanole is removed at reduced pressure on water bath.

The residual is mixed with 1 lt. of water and the organic phase is extracted with chloroform many times.

Chloroformic extracts together are washed with water and dehydrated on sodium sulphate anhydre, the solvent is distilled and the residual christallized with methanole gives compound (V) with 35-40% yield of calculated and thr product is the same as obteined from method A and B in white, soft, bright chri

stals, m.p. 69.5-70°C, in Kofler.

METHOD D:

1) 2-Mercaptoethyl-nicotinate (III) (0.2 mole) .........gr 36.8

2) 1.9-Decadiene (VI) (0.1 mole) .......................gr 13.9

Compounds 1) and 2) together, while stirring, are radiated for about 2 hrs. at 20 cm. of distance, by a 360 Watt mercury lamp.

The reaction mixture is dissolved in chloroform and washed several times with water, dehydrated on sodium sulphate anhidrous and filtered. The solvent is evaporated in vacuum on water bath and the residual still hot is seeded with some crystals of the product and cooled in refrigerator all the night.

The product obteined, chrystallized with ethanole is the same product obteined from methods A, B and C, with yield 35% of theoricum.

I CLAIM:

1) 2-(Decamethylendithio)-2'-diethyl-pyriden-3-carboxilate

or

2-(Decamethylendithio)-2'-diethyldinicotinate of the Structural formula:

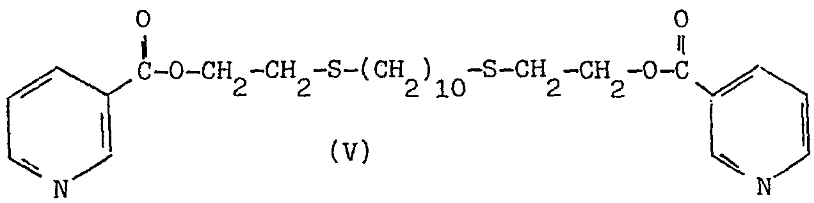

(V)

wherein the aliphatic radical $-(CH_2)_{10}-$may also be a different number of the atoms of carbon $-(CH_2)_n-$

2) The compound $-R-CH_2-CH_2-S-CH_2-CH_2-R$, wherein R may be an acylic radical of an aromatic acid, arylalkyl, etherociclic or aliphatic, with a hypolipidemizing activity.

3) The compound (V) of claim 1)- obteined according to Method A from the clorure of the acid with (bis-hydroxyethyl-thio)--1.10-decane in presence of an accepter of hydrochloric acid, such as Pyridine, Triethylamine, N.N-Dimethylniline, etc. and a solvent, as chloroform, methylene chloride, benzene, toluene.

4) The product (V) of claim 1) - obteined according to Method B by reaction of anhidride of nicotinic acid with 1.10 (bis-hy=droxyethyl-thio)-decane in presence of Pyridine or by reaction of nicotinic acid with 1.10-(bis-hydroxiethyl-thio)-decane in

presence of Strong Cationic Resin, for exaple Duolite C - - 20 at suitable temperature.

5) The product (V) of claim 1) - obteined according to Method C by reaction of nicotinylchloride or its anhidride with 2-Mercaptoethylnicotinate (III) and further reaction with 1.10-Dibromodecane (IV) in presence of hydrosoluble Solvent.

6) The product III, which is an intermediate for the production of product (V) according to Method C.

7) The product III B, thioesther, prepared according to claim 6, with a periferic vessel-dilatation activity.

8) The product (V) of claim 1)- obteined according to Method D by reaction of 2-Mercaptoethyl nicotinate (III) with 1.9-Decadiene (VI) in presence of 360 Watt mercury discharge - lamp for about two hours.

9) A pharmaceutical preparation including the compound of claim 1) and 2) or a mixture containing two or more compounds for oral administrations, hypodermic injection and retard form in human subjects.

Celesio Sonnino TAURO

Celesio Sonnino Tauro

0053681

European Patent Office

# EUROPEAN SEARCH REPORT

Application number

EP 81 10 8526

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| X | FR - A - 2 146 138 (L. LAFON)<br>* Claims; example 2; page 1, lines 32-34 *<br>-- | 1-3,9 | C 07 D 213/80<br>213/83<br>A 61 K 31/455 |
| X | CHEMICAL ABSTRACTS, vol. 89, no. 15, October 9, 1978, abstract 129060m, page 549<br>Columbus, Ohio, US<br>A. GALLARDO-CARRERA: "1,10-Bis (β -hydroxyethylthio) decane derivatives"<br>& ES - A - 456 787 (A. GALLARDO S.A.)(16-01-1978)<br>* Abstract *<br>-- | 1,2,9 | TECHNICAL FIELDS SEARCHED (Int.Cl. 3)<br>C 07 D 213/00<br>A 61 K 31/00 |
| X | CHEMICAL ABSTRACTS, vol. 89, no. 15, October 9, 1978, abstract 129411b page 581<br>Columbus, Ohio, US<br>A. GALLARDO-CARRERA: "ω-Disubstituted decane derivative"<br>& ES - A - 456 788 A. GALLARDO S.A.)( 01-02-1978)<br>* Abstract *<br>-- | 1-3,9 | |
| Y | US - A - 4 021 436 (C. CAVAZZA)<br>* The whole document *<br>-- | 1-3,9 | CATEGORY OF CITED DOCUMENTS<br>X: particularly relevant if taken alone<br>Y: particularly relevant if combined with another document of the same category<br>A: technological background<br>O: non-written disclosure<br>P: intermediate document<br>T: theory or principle underlying the invention<br>E: earlier patent document, but published on, or after the filing date<br>D: document cited in the application<br>L: document cited for other reasons |
| Y | FR - A - 2 324 303 (SIGMA-TAU)<br>* The whole patent *<br>--<br>./. | 1,2,9 | |
| X | The present search report has been drawn up for all claims | | &: member of the same patent family, corresponding document |

| Place of search | Date of completion of the search | Examiner |
|---|---|---|
| The Hague | 19-03-1982 | NUYTS |

EPO Form 1503.1 06.78

0053681

European Patent Office

**EUROPEAN SEARCH REPORT**

Application number
EP 81 10 8526



## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| Y | FR - A - 2 195 433 (SIGMA-TAU)<br>* The whole document *<br>---- | 1-3,6,9 | |
| | | | TECHNICAL FIELDS SEARCHED (Int. Cl.3) |